# EUROPÄISCHE PATENTANMELDUNG

(11) **EP 2 308 398 A1**
(43) Veröffentlichungstag der Anmeldung: **13.04.2011**
(21) Anmeldenummer: 10011391.9
(22) Anmeldetag: 29.09.2010
(51) Int. Cl.: A61B 17/225, A61B 17/00

(54) **Medizinische Therapieeinrichtung**

(30) Priorität: 06.10.2009 DE 102009048361
(71) Anmelder: Richard Wolf GmbH, 75438 Knittlingen (DE)
(72) Erfinder: Burkhardt, Michael, 75417 Mühlacker (DE); Messina, Francesco, 76703 Kraichtal (DE); Zimmermann, Jean-Georges, 75438 Knittlingen (DE)
(74) Vertreter: Vollmann, Heiko

(57) **Zusammenfassung**

Die medizinische Therapieeinrichtung weist mindestens eine bildgebende Ortungseinrichtung (5, 6; 8) eine Behandlungseinrichtung (3, 4), eine Patientenauflage (2), sowie Mittel zum motorischen Positionieren mindestens der Behandlungseinrichtung (3, 4) relativ zur Patientenauflage (2) sowie Mittel zum Steuern der Therapieeinrichtung und eine Anzeige- und Bedieneinrichtung (10) auf. Auf der Anzeige ist das Behandlungszentrum der Behandlungseinrichtung (3, 4) in mindestens einem Bild der Ortungseinrichtung (5, 6; 8) darstellbar und es sind Bedienelemente vorgesehen sind, mit denen die Positionierung in Koordinaten der Ortungseinrichtung (5, 6; 8) steuerbar ist.

## Beschreibung

Die Erfindung betrifft eine medizinische Therapieeinrichtung, insbesondere eine Schallwellenbehandlungseinrichtung sowie eine Anzeige- und Bedieneinrichtung für eine solche Einrichtung.

Derartige Einrichtungen zählen zum Stand der Technik und bestehen typischerweise aus einer bildgebenden Ortungseinrichtung, einer Behandlungseinrichtung, insbesondere einer fokussierenden Schallwellenbehandlungseinrichtung sowie einer Patientenauflage, die motorisch verfahrbar ist, so dass die zu behandelnde Stelle im Patientenkörper, z. B. ein Konkrement, in Übereinstimmung mit dem Behandlungszentrum der Behandlungseinrichtung, also mit dem Fokus der Schallwellenbehandlungseinrichtung gebracht werden kann. Weiterhin sind Mittel zum Steuern der Therapieeinrichtung und eine Anzeige- und Bedieneinheit vorgesehen. Die Steuermittel dienen dabei einerseits zur Aktivierung der Behandlungseinrichtung andererseits zum Positionieren der Patientenauflage. Hierzu ist eine Anzeige vorgesehen, welche ein Bild der Ortungseinrichtung darstellt sowie darüber hinaus den Fokus der Schallwellenbehandlungseinrichtung. Als Ortungseinrichtungen sind typischerweise einerseits eine Ultraschallortungseinrichtung und andererseits eine Röntgenortungseinrichtung vorgesehen, wobei für jede der Ortungseinrichtungen eine gesonderte Anzeige vorgesehen ist. Eine solche Einrichtung ist beispielsweise aus US 2008/0146908 A1 bekannt.

Nachteilig bei einer solchen Behandlungseinrichtung ist, dass eine Orientierung auf der Anzeige der Ortungseinrichtung zur tatsächlichen Lage des Patienten bzw. des zu behandelnden Ziels im Patienten häufig schwierig ist, da die Anzeige an einer bestimmten Stelle stationär neben der typischerweise als Liege ausgebildeten Patientenauflage angeordnet ist. Die Patientenauflage ist dabei in Tischkoordinaten verfahrbar also wie bei derartigen Tischen üblich, hoch - runter, links - rechts und vor - zurück. Diese Richtungen stimmen jedoch typischerweise nicht mit denen in der Anzeigedarstellung der Ortungssysteme überein, weshalb der Therapeut zunächst durch Probieren herausfinden muss, durch welche Tischverstellung sich der Fokus der Schallwellenbehandlungseinrichtung in Richtung zum Zielpunkt im Patienten bewegen lässt. Dies kann dazu führen, dass der Zielpunkt, also beispielsweise das zu behandelnde Konkrement durch Verfahren der Patientenauflage in die falsche Richtung im Bild verloren geht, was eine Neuausrichtung und somit kostbare Zeit kostet. Auch führt dies dazu, dass häufig eine Zielkontrolle während der Behandlung unterbleibt.

Aus DE 10 2006 060 070 A1 zählt es zwar schon zum Stand der Technik, bei einer Lithotripsie-Einrichtung mittels eines aufwendigen Raumnavigationssystems die Position der Schallwellenbehandlungseinrichtung in Bezug auf die Patientenauflage in raumfesten Koordinaten zu erfassen, um auf diese Weise auch eine automatische Positionierung des Zielpunktes in Bezug auf den Fokus der Schallwellenbehandlungseinrichtung zu erzielen, die übliche und bei der Behandlung auch regelmäßig erforderliche manuelle Steuerung bzw. Nachkorrektur erfolgt jedoch in gleicher nachteiliger Weise wie vorbeschrieben.

Vor diesem Hintergrund liegt der Erfindung die Aufgabe zugrunde, eine medizinische Therapieeinrichtung, insbesondere der vorbeschriebenen Art so auszubilden, dass die Bedienung vereinfacht und somit sicherer wird, insbesondere im Hinblick auf die Positionierung des im Patientenkörper befindlichen Zielpunktes in das Behandlungszentrum der Behandlungseinrichtung, also z. B. in den Fokus der Schallwellenbehandlungseinrichtung.

Diese Aufgabe wird gemäß der Erfindung durch die in Anspruch 1 angegebenen Merkmale gelöst, insbesondere durch eine Anzeige- und Bedieneinheit gemäß Anspruch 12. Vorteilhafte Ausgestaltung der Erfindung sind in den Unteransprüchen, der nachfolgenden Beschreibung und der Zeichnung angegeben.

Die medizinische Therapieeinrichtung gemäß der Erfindung weist mindestens eine bildgebende Ortungseinrichtung, eine Behandlungseinrichtung und eine Patientenauflage auf. Sie weist weiter Mittel zum motorischen Positionieren mindestens der Behandlungseinrichtung relativ zur Patientenauflage auf sowie darüber hinaus Mittel zum Steuern der Therapieeinrichtung sowie eine Anzeige- und Bedieneinrichtung, auf der das Behandlungszentrum der Behandlungseinrichtung in mindestens einem Bild der Ortungseinrichtung darstellbar ist. Gemäß der Erfindung sind an der Anzeige- und Bedieneinrichtung Bedienelemente vorgesehen, mit denen die Positionierung in Koordinaten der Ortungseinrichtung steuerbar ist.

Erfindungsgemäß ist eine Anzeige- und Bedieneinrichtung, vorzugsweise in Form einer Anzeige- und Bedieneinheit vorgesehen, die eine Anzeige aufweist, auf der mindestens ein Bild einer Ortungseinrichtung in Bezug auf ein Behandlungszentrum einer Behandlungseinrichtung dargestellt ist bzw. darstellbar ist und die weiterhin Bedienelemente aufweist, mit denen das Behandlungszentrum in den Koordinaten des Bildes der Ortungseinrichtung zum Zwecke der Positionierung des Patienten verstellbar ist.

Die erfindungsgemäße Lösung ist grundsätzlich bei unterschiedlichsten medizinischen Therapieeinrichtungen einsetzbar, besonders vorteilhaft jedoch bei Therapieeinrichtungen in Form von extrakorporalen Schallwellenbehandlungseinrichtungen, bei denen die Behandlungseinrichtung eine fokussierende Schallwellenbehandlungseinrichtung ist und das Behandlungszentrum durch den Fokus der Schallwellenbehandlungseinrichtung gebildet ist, also an solchen Therapieeinrichtungen, wie sie aus dem einleitend genannten Stand der Technik bekannt sind. Dabei erfolgt die Positionierung typischerweise durch eine entsprechende Relativbewegung zwischen der Patientenauflage und mindestens der Behandlungseinrichtung, typischerweise jedoch der Behandlungseinrichtung zusammen mit einer oder mehrerer Ortungseinrichtungen. Grundsätzlich spielt es dabei keine Rolle, ob die Behandlungseinrichtung zur Patientenauflage oder die Patientenauflage zur Behandlungseinrichtung hin bewegt wird. Üblicherweise erfolgt die Positionierung aber durch entsprechendes Verfahren der Patientenauflage.

Grundgedanke der vorliegenden Erfindung ist es somit, die Positionierung zwischen Behandlungseinrichtung und Patientenauflage, die typischerweise durch Verfahren der Patientenauflage in Tischkoordinaten (hoch - runter, rechts - links und vor - zurück) erfolgt, nicht mehr in diesen Tischkoordinaten anzusteuern, sondern in den Bildkoordinaten der Ortungseinrichtung, also in den Koordinaten der Anzeige der Ortungseinrichtung bzw. der Ortungseinrichtungen, wenn mehrere vorgesehen sind. Der behandelnde Arzt kann somit durch die Bedienelemente typischerweise vier oder sechs Bedienelemente zur Bewegung in zwei bzw. drei Raumkoordinaten die Positionierung entsprechend den auf der Anzeige sichtbaren Koordinaten der Ortungseinrichtung durchführen, braucht sich also über die Umrechnung der Anzeigekoordinaten in Tischkoordinaten keinerlei Gedanken zu machen, sondern kann zielgerichtet den Patienten auf kürzestem Wege derart positionieren, dass der Zielpunkt im Körper, beispielsweise das Konkrement in Übereinstimmung mit dem Fokus der Schallwellenbehandlungseinrichtung gebracht wird. Dabei spielt es grundsätzlich keine Rolle, ob die Behandlungseinrichtung relativ zum Patienten oder der Patient relativ zur Behandlungseinrichtung bewegt wird, in der Praxis hat es sich allerdings bewährt, die Patientenauflage motorisch zu verfahren. Um den Fokus der Schallwellenbehandlungseinrichtung in Richtung zum Zielpunkt im Körper zu bewegen, also die vorbeschriebene Positionierung durchzuführen, muss der Therapeut lediglich entsprechend markierte Bedienelemente bedienen, an welchen die Bewegungsrichtung so dargestellt ist, wie sie beim Bewegen des Fokus auf dem angezeigten Bild in Richtung zum Zielpunkt erforderlich wäre. Liegt also beispielsweise der Zielpunkt bezogen auf den Fokus im angezeigten Bild im rechten oberen Bildviertel, so sind die Bedienelemente zu betätigen, welche bezogen auf das dargestellte Bild nach rechts und nach oben weisen, bis Übereinstimmung besteht.

Durch die erfindungsgemäße Ausbildung erfolgt nicht nur eine wesentlich schnellere Positionierung als beim Stand der Technik, sondern auch wesentlich sicherer und zuverlässiger in die gewünschte Richtung, weshalb die Gefahr, dass das Konkrement aus dem Bild der Anzeige verloren geht, praktisch nicht mehr besteht. Die erfindungsgemäße Lösung erlaubt zudem auch während der Behandlung stets eine Kontrolle und Korrektur, sofern sich Zielpunkt und Fokus, z. B. aufgrund der Atembewegung des Patienten, verschoben haben.

Vorteilhaft wird als Ortungseinrichtung eine Ultraschallortungseinrichtung vorgesehen, deren Bild mindestens auf der Anzeige der Anzeige- und Bedieneinrichtung dargestellt ist. Es versteht sich, dass zusätzlich das Bild dieser Ortungseinrichtung auch wie beim Stand der Technik üblich auf einer gesonderten Anzeige dargestellt sein kann.

Gemäß einer Weiterbildung der Erfindung ist als Ortungseinrichtung alternativ oder vorteilhaft zusätzlich eine Röntgenortungseinrichtung, vorzugsweise ein Röntgen-C-Bogen vorgesehen, deren Bild mindestens auf der Anzeige der Anzeige- und Bedieneinrichtung dargestellt ist. Auch hier versteht es sich, dass eine Darstellung des Bildes auf einem weiteren Bildschirm möglich ist. Da insbesondere ein Röntgen-C- Bogen auch anderweitig einsetzbar, kann es insoweit sinnvoll sein, die Darstellung auf der Anzeige der Anzeige- und Bedienreinrichtung gemäß der Erfindung zusätzlich neben der üblichen Darstellung auf einen Monitor vorzusehen.

Insbesondere bei Lithotripsie-Vorrichtungen ist es vorteilhaft, sowohl eine Ultraschallortungseinrichtung als auch eine Röntgenortungseinrichtung vorzusehen. Die Bilder der Ortungseinrichtungen sollten zweckmäßigerweise nebeneinander jeweils mit Darstellung des Fokus der Schallbehandlungseinrichtung gleichzeitig auf der Anzeige der Anzeige- und Bedieneinrichtung dargestellt sein. Da die vorgenannten Ortungseinrichtungen sowohl hinsichtlich der räumlichen Lage als auch hinsichtlich der Darstellung unterschiedliche Informationen geben ist es besonders sinnvoll, die Bilder beider Ortungseinrichtungen nebeneinander auf der Anzeige vorzusehen wobei dann zweckmäßigerweise jeder Bilddarstellung entsprechende Bedienelemente zur Positionierung zugeordnet sind die den Koordinaten des jeweiligen Bildes entsprechen

Die Anzeige- und Bedieneinrichtung weist vorteilhaft gemäß einer Weiterbildung der Erfindung mindestens einen Sensorbildschirm zur Anzeige des Bildes oder der Bilder der Ortungseinrichtung/en auf und beinhaltet darüber hinaus mindestens die Bedienelemente zur Positionierung. Eine solche Ausbildung ist besonders vorteilhaft, da die Bedienelemente bildnah oder im Bild angeordnet werden können und die Positionierung durch manuelle Ansteuerung mittels der Bedienelemente sich quasi intuitiv ergibt und zudem im aktuellen Bild sichtbar ist. Vorteilhaft sind auf dem Sensorbildschirm nicht nur die Bedienelemente zur Positionierung sondern auch alle übrigen Bedienelemente der Therapieeinrichtung übersichtlich angeordnet, da dann die gesamte Bedienung der Einrichtung einschließlich Ortung- und Zielführung mittels des Sensorbildschirms erfolgen können.

Dabei sind die Bedienelemente als Tastenfelder ausgebildet, die ohne weitere Instrumente vorteilhaft nur durch Fingerberührung ausgelöst werden können. Soweit es die Bedienelemente zur Positionierung angeht ist es vorteilhaft, die entsprechenden Tastenfelder als Pfeiltastenfelder auszubilden und an den seitlichen Rändern der jeweiligen Bilddarstellung auf dem Sensorbildschirm anzuordnen. Durch Berühren der Pfeiltasten erfolgt dann eine Positionierung, und zwar der Gestalt, dass ein den Zielfokus des Schallwellenbehandlungsgerätes repräsentierendes Zielkreuz durch Berühren der entsprechenden Pfeiltasten in Überdeckung mit dem Ziel im Bild der Ortungseinrichtung gebracht wird. Dabei bleibt das Zielkreuz typischerweise in der Mitte des Ortungsbildes stehen während das Ziel durch Betätigung der entsprechenden Pfeiltasten über das Bild wandert, also der Patient relativ zum Therapie- und Ortungsgerät positioniert wird. Während beim Bild der Röntgenortungseinrichtung vorteilhaft vier Pfeile an dem Bildrändern zur zweidimensionalen Verschiebung angeordnet sind ist an den Bildrändern der Ultraschallortungseinrichtung zusätzlich zu diesen vier Pfeilen vorteilhaft an zwei Eckpunkten je eine diagonal verlaufende Pfeiltaste vorgesehen, welche eine Positionierung in Tiefrichtung also in der dritten Dimension des Bildes ermöglicht.

Es versteht sich, dass die Tastenanordnungen den jeweiligen Bildkoordinaten zugeordnet sind, d. h. dass beispielsweise beim Betätigen eines Bedienelements im Bild der Ultraschallortungseinrichtung nach unten ein hoch bzw. runterfahren des Patiententisches bewirkt, wohingegen eine Betätigung des gleichgerichteten Pfeils auf dem Bild der Röntgenortungseinrichtung daneben ein Verfahren des Tisches in einer horizontalen Richtung bewirkt. Je nach Anordnung der Ortungseinrichtungen kann dies jedoch auch gleichsinnig sein.

Eine besonders praktische Handhabung ergibt sich, wenn die Anzeige- und Bedieneinrichtung als Einheit ausgebildet ist, insbesondere durch einen vorzugsweise kabellosen Sensorbildschirm gebildet ist. Dieser ermöglicht eine Bedienung aus jeder Lage des Therapeuten, gegebenenfalls sogar geschützt hinter einer röntgen-abgeschirmten Wand.

Zur Positionierung ist es besonders vorteilhaft, die Patientenauflage motorisch in drei Raumkoordinaten verfahrbar auszugestalten, die Umrechnung in Bildkoordinaten kann dann softwaremäßig in der Steuerung erfolgen.

Mit der erfindungsgemäßen Therapieeinrichtung ist nicht nur eine manuelle halbautomatische Positionierung des Patienten relativ zur Behandlungseinrichtung möglich sondern gemäß einer Weiterbildung der Erfindung auch eine selbsttätige Positionierung des Zentrums der Behandlungseinrichtung, insbesondere des Fokus der Schallwellenbehandlungseinrichtung auf ein zuvor auf der Anzeige bestimmtes Ziel vorgesehen, also ein automatischer Modus zur Zielführung. Hierzu markiert der Therapeut nach Aktivierung z. B. der Zielführung zunächst typischerweise mit einem Marker (dies kann ein zweites Zielkreuz, ein Stift oder der Finger des Anwenders sein) auf einem der beiden Bilder der Ortungseinrichtungen das Zielgebiet im Patienten, also beispielsweise das Konkrement, wonach der automatische Modus aktiviert wird und selbsttätig die Positionierung erfolgt, In diesem automatischen Modus kann auch während der Behandlung eine selbsttätige Zielnachführung erfolgen.

Die Erfindung ist nachfolgend anhand von in den Figuren dargestellten Ausführungsbeispielen näher erläutert. Es zeigen
- Fig. 1: in vereinfachter schematischer Darstellung eine Therapie- einrichtung gemäß der Erfindung,
- Fig. 2: die Ausgestaltung eines Sensorbildschirms gemäß der Er- findung,
- Fig. 3: eine alternative Ausgestaltung des Sensorsbildschirms für die Darstellung des Bildes einer Ultraschallortungseinrich- tung und
- Fig. 4: eine weitere Alternative für die Darstellung des Bildes einer Röntgenortungseinrichtung.

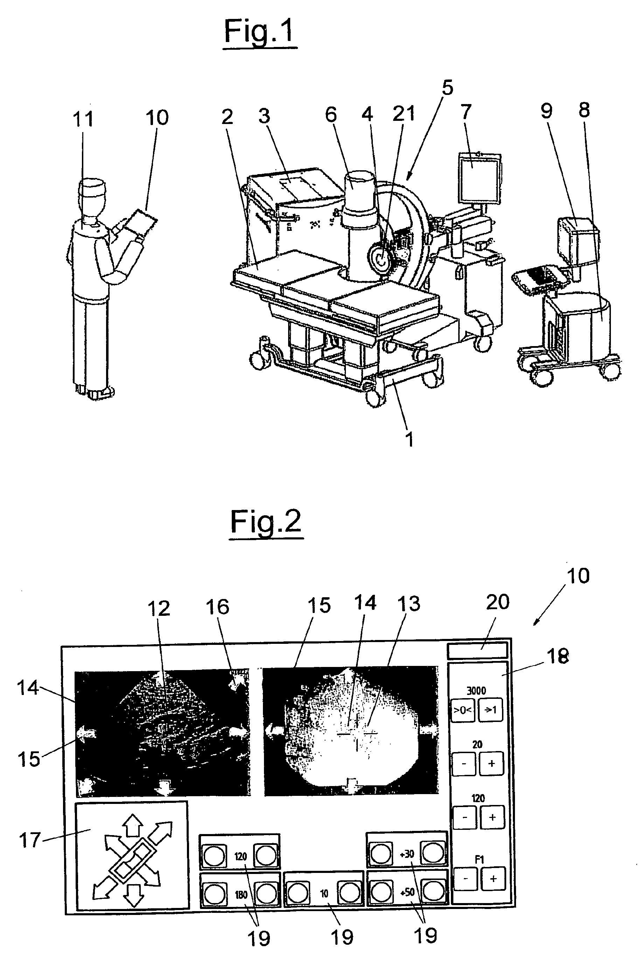

Die anhand von Fig. 1 dargestellte Therapieeinrichtung ist eine Lithotripsie-Einrichtung mit einer Patientenliege 1 deren Auflage 2, auf der der Patient während der Behandlung liegt, elektromotorisch in drei Raumkoordinaten verfahrbar ist. Als Behandlungseinrichtung ist ein Lithotriptor 3 vorgesehen, dessen fokussierende Schallwellen erzeugender Behandlungskopf 4 neben der in diesem Bereich ausgesparten Auflage 2 der Patientenliege 1 schwenkbar angeordnet ist. Weiterhin vorgesehen ist eine Röntgenortungseinrichtung 5, dessen Röntgenbildverstärker 6 in der Darstellung gemäß Fig. 1 über der Auflage 2 mit Abstand angeordnet ist. Die Röntgenortungseinrichtung 5 ist mechanisch mit dem Behandlungskopf 4 des Lithotriptor 3 gekoppelt und kann in an sich bekannter Weise um eine horizontale Achse geschwenkt werden um eine dreidimensionale Ortung zu ermöglichen. Das Bild der Röntgenortungseinrichtung 5 ist auf einem Röntgenmonitor 7 darstellbar. Weiterhin ist ein Ultraschallortungsgerät 8 vorgesehen, dessen Ortungskopf 21 innerhalb des Behandlungskopfes 4 angeordnet ist und das einen Ultraschallmonitor 9 zur Darstellung des Ultraschallbildes aufweist.

Die vorbeschriebenen Teile 1 bis 9 der Einrichtung einschließlich der zugehörigen Steuerelektronik und Bedieneinrichtungen ist an sich bekannt (US 2008/0146908A1) und wird deshalb hier nicht im Einzelnen beschrieben.

Die anhand von Fig. 1 dargestellte Therapieeinrichtung ist mittels einer Anzeige- und Bedieneinheit in Form eines Sensorbildschirms 10 (Touchscreen) bedienbar, der über Funk an die Steuerung der Therapieeinrichtung, insbesondere auch die Ortungseinrichtungen angebunden ist. Der hier beispielhaft dargestellte Therapeut 11, der den Sensorbildschirm 10 hält, kann sämtliche behandlungswesentlichen Funktionen der Einrichtung mittels des Sensorbildschirms 10 steuern und gleichzeitig die Bilder 12, 13 beider Ortungseinrichtungen, nämlich der Röntgenortungseinrichtung 5 und der Ultraschallortungseinrichtung 8 nebeneinander sehen.

Das Bild 12 der Ultraschallortungseinrichtung 8 ist links neben dem Bild 13 der Röntgenortungseinrichtung 5 auf dem Sensorbildschirm 10 dargestellt. In beiden Bildern 12, 13 ist der Fokus des Behandlungskopfes 4 in Form eines Zielkreuzes 14 im Bild dargestellt. An den Rändern jeder der Bilder 12 und 13 sind jeweils vier Pfeiltasten 15 vorgesehen, die berührungsempfindlich sind und beim Berühren eine Positionierung der Auflage 2 der Patientenliege 1 in Bezug auf den durch das Zielkreuz 14 repräsentierten Fokuspunkt der Behandlungseinrichtung 3, 4 bewirken. Dabei ist die Steuerung so, dass die Pfeiltasten 15 entsprechend den Bildkoordinaten der Bilder 12 und 13 ausgerichtet sind, so dass das jeweilige Bild mit dem darin befindlichen Zielpunkt quasi unter dem Zielkreuz 14 entsprechend den Richtungen der jeweiligen Pfeiltasten 15 geschoben wird, wodurch die Patientenauflage 2 mit dem darauf befindlichen Patienten entsprechend positioniert wird. Dies erfolgt solange bis der Therapeut eine Übereinstimmung zwischen Zielkreuz 14 und dem Ziel im Körper des Patienten erreicht hat.

Da die Pfeiltasten 15 in beiden Bildern 13 und 14 eine Positionierung in nur zwei Dimensionen ermöglichen sind in den Ecken des Bildes 12 der Ultraschallortungseinrichtung 8 diagonal angeordnete Pfeiltasten 16 vorgesehen, mit denen eine Positionierung in der dritten Dimension also in einer Richtung senkrecht zur Bildebene des Sensorbildschirms 10 erfolgen kann.

Die Pfeiltasten 15 und 16 sind in an sich bekannter Weise als berührungsempfindliche Tasten ausgebildet, die Anordnung erfolgt durch entsprechende Softwareansteuerung des Sensorbildschirms 10. Unabhängig von den Pfeiltasten 15 und 16, welche eine Positionierung des Patienten unter Zugrundelegung der Ortungsbilder 12 und 13 relativ zum Fokus 14 ermöglichen, ist ein Bedienfeld 17 vorgesehen, mit welchen die Patientenauflage in Tischkoordinaten verfahrbar ist, so wie es zu einem Grobausrichten des Patienten zum Auffinden des Zielgebiets zweckmäßig ist. Die dort dargestellten sechs Pfeiltasten sind ebenfalls als berührungsempfindliche Bedienfelder ausgebildet und ermöglichen eine Positionierung der Auflage 2 nach vorne, hinten, links, rechts, oben und unten.

Weiterhin ist am rechten Rand des Sensorbildschirms 10 ein Bedienfeld 18 vorgesehen, welches das Stoßwellenbedienfeld bildet, mit dem der Therapiekopf 4 zur Behandlung angesteuert werden kann. Weitere Bedienfelder 19 für unterschiedliche Funktionen der Einrichtung sind ebenfalls auf dem Sensorbildschirm 10 vorgesehen um so eine einheitliche Bedienung auf einem Bildschirm zu gewährleisten.

Neben der vorbeschriebenen halbautomatischen Positionierung des Patienten bzw. der Auflage 2 in Bezug auf den Fokus des Therapiekopfs 4 besteht die Möglichkeit durch Aktivierung eines automatischen Modus einer selbsttätige Positionierung auszulösen. Hierzu ist zunächst ein Tastenfeld 20 zu aktivieren, wonach das Zielgebiet in einem oder beiden den der Ortungsbilder 12 und 13 mittels eines Markers (zweites Zielkreuz, Stift oder der Finger des Anwenders), markiert und nach Betätigung einer Auslösungstaste aktiviert wird. Es erfolgt dann nach entsprechender Berechnung selbsttätig eine automatische Positionierung derart, dass die auf dem Bild 12 oder 13 markierte Stelle in Übereinstimmung mit dem Zielkreuz 14, also dem Fokus der Therapiequelle gebracht wird.

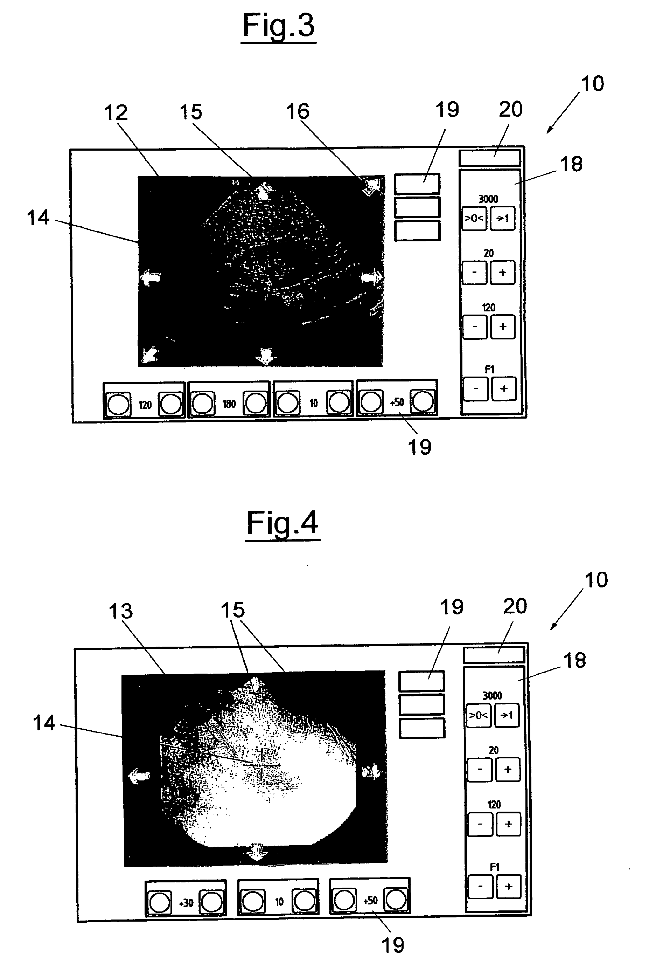

Anhand der Fig. 3 und 4 ist ein Sensorbildschirm 10 dargestellt, welcher einer Therapieeinrichtung zugeordnet ist, die wahlweise ein Ultraschallortungsbild 12 (Fig. 3) oder ein Röntgenortungsbild 13 (Fig. 4)anzeigt. Die Positionierung erfolgt in gleicher Weise wie vorbeschrieben, sie kann ebenfalls im Automodus oder halbautomatisch also durch Betätigung der Pfeiltasten 15, 16 durch den Therapeuten 11 erfolgen. Es versteht sich, dass auch bei der Verwendung nur eines Ortungssystems, wie es anhand der Figuren 3 bzw. 4 dargestellt ist, weitere Bedienfelder 17, 18, 19, wie beispielsweise das Bedienfeld 17 zur Steuerung der Auflage 2 in Raumkoordinaten oder andere Funktionen integriert werden können.

Wie die Ausführungsvarianten nach den Fig. 2 bis 4 verdeutlichen, kann mittels des Sensorbildschirms 10 (Touchscreen) nicht nur in einfachster Weise eine In-Bild-Navigation erfolgen, sondern es können darüber hinaus auch praktisch beliebige Bedienfunktionen vorgesehen werden. Dabei können das oder die Ortungsbilder 12, 13 an geeigneter Stelle und in geeigneter Größe auf dem Bildschirm dargestellt werden, ebenso die Bedienfelder 17 bis 19. Auch kann gegebenenfalls eine erste Darstellung auf dem Bildschirm 10 ausschließlich zur In-Bild-Navigation und eine zweite Darstellung mit den entsprechend zugeordneten Bedienfeldern 17 bis 19 erfolgen, wobei lediglich eine Umschaltfunktion am Bildschirm 10 vorzusehen ist. Auch können die Bedienfelder 17 bis 19 an praktisch beliebige Therapieeinrichtungen in geeigneter Weise angepasst werden. Es können dem Anwender unterschiedliche Darstellungsarten/Farben/Gruppierungen angeboten werden, so dass der Therapeut sich die Darstellung auf dem Bildschirm 10 quasi individuell nach seinen Bedürfnissen einrichten kann. Die entsprechenden Anordnungen und Darstellungen können auf einfache Weise softwaremäßig implementiert werden, so dass auch Änderungen durchführbar sind, wenn beispielsweise ein Gerät der Therapieeinrichtung durch ein anderes ersetzt wird.

### Bezugszeichenliste

- 1 -: Patientenliege
- 2 -: Patientenauflage
- 3 -: Lithotriptor
- 4 -: Behandlungskopf von 3
- 5 -: Röntgenortungsgerät
- 6 -: Röntgenbildverstärker
- 7 -: Röntgenmonitor
- 8 -: Ultraschallortungsgerät
- 9 -: Ultraschallmonitor
- 10 -: Sensorbildschirm
- 11 -: Therapeut
- 12 -: Bild der Ultraschallortungseinrichtung
- 13 -: Bild der Röntgenortungseinrichtung
- 14 -: Zielkreuz (Therapiefokus)
- 15 -: Pfeiltasten am Bildrand
- 16 -: Pfeiltasten in den Bildecken
- 17 -: Bedienfeld
- 18 -: Bedienfeld
- 19 -: Bedienfeld
- 20 -: Tastenfeld
- 21: Ultraschallortungssonde

## Patentansprüche

1. Medizinische Therapieeinrichtung mit mindestens einer bildgebenden Ortungseinrichtung (5, 6; 8, 21), mit einer Behandlungseinrichtung (3, 4) und mit einer Patientenauflage, mit Mitteln zum motorischen Positionieren mindestens der Behandlungseinrichtung (3, 4, 6) relativ zur Patientenauflage (2), mit Mitteln zum Steuern der Therapieeinrichtung und mit einer Anzeige- und Bedieneinrichtung (10), auf der das Behandlungszentrum (14) der Behandlungseinrichtung (3, 4) in mindestens einem Bild (12,13) der Ortungseinrichtung (5, 6; 8, 21) darstellbar ist, und an der Bedienelemente (15, 16) vorgesehen sind, mit denen die Positionierung in Koordinaten der Ortungseinrichtung (5, 6; 8, 21) steuerbar ist.

2. Therapieeinrichtung nach Anspruch 1, die eine Lithotripsie-Einrichtung ist, bei der die Behandlungseinrichtung eine fokussierende Schallwellenbehandlungseinrichtung (3, 4) ist und das Behandlungszentrum (14) durch den Fokus der Schallwellenbehandlungseinrichtung (3, 4) gebildet ist.

3. Therapieeinrichtung nach Anspruch 1 oder 2, bei der als Ortungseinrichtung eine Ultraschallortungseinrichtung (8) vorgesehen ist, deren Bild (12) mindestens auf der Anzeige (10) der Anzeige- und Bedieneinrichtung dargestellt ist.

4. Therapieeinrichtung nach einem der vorhergehenden Ansprüche, bei der als Ortungseinrichtung eine Röntgenortungseinrichtung (5, 6), vorzugsweise ein Röntgen-C-Bogen (5) vorgesehen ist, deren Bild (13) mindestens auf der Anzeige (10) der Anzeige- und Bedieneinrichtung dargestellt ist.

5. Therapieeinrichtung nach einem der vorhergehenden Ansprüche, bei der die Bilder (12, 13) zweier Ortungseinrichtungen (5, 8) nebeneinander jeweils mit dem Fokus (14) der Schallwellenbehandlungseinrichtung (3, 4) gleichzeitig auf der Anzeige (10) der Anzeige- und Bedieneinrichtung dargestellt sind.

6. Therapieeinrichtung nach einem der vorhergehenden Ansprüche, bei der die Anzeige- und Bedieneinrichtung mindestens einen Sensorbildschirm (10) zur Anzeige des Bildes oder der Bilder (12, 13) der Ortungseinrichtung/en (5, 6, 8) aufweist und der mindestens die Bedienelemente (15, 16) zur Positionierung beinhaltet.

7. Therapieeinrichtung nach einem der vorhergehenden Ansprüche, bei der die Bedienelemente zur Positionierung durch Tastenfelder (15, 16), insbesondere Pfeiltastenfelder an den seitlichen Rändern der jeweiligen Bilddarstellung (12, 13) auf dem Sensorbildschirm (10) gebildet sind.

8. Therapieeinrichtung nach einem der vorhergehenden Ansprüche, bei der die Anzeige- und Bedieneinrichtung durch einen vorzugsweise kabellosen Sensorbildschirm (10) gebildet ist.

9. Therapieeinrichtung nach einem der vorhergehenden Ansprüche, bei der die Patientenauflage (2) motorisch in drei Raumkoordinaten verfahrbar ist.

10. Therapieeinrichtung nach einem der vorhergehenden Ansprüche, bei der in einem automatischen Modus eine selbsttätige Positionierung des Zentrums (14) der Behandlungseinrichtung (3, 4), insbesondere des Fokus der Schallwellenbehandlungseinrichtung, auf ein zuvor auf der Anzeige bestimmtes Ziel erfolgt.
